(19) Europäisches Patentamt
European Patent Office
Office européen des brevets

(11) Publication number: 0 139 477
A2

(12) # EUROPEAN PATENT APPLICATION

(21) Application number: 84306363.7

(22) Date of filing: 18.09.84

(51) Int. Cl.⁴: C 07 D 239/42
C 07 D 239/34

(30) Priority: 23.09.83 US 535199

(43) Date of publication of application: 02.05.85 Bulletin 85/18

(84) Designated Contracting States: AT BE CH DE FR GB IT LI LU NL SE

(71) Applicant: ELI LILLY AND COMPANY
307, East McCarty Street
Indianapolis Indiana 46285(US)

(72) Inventor: Kress, Thomas Joseph
215 East Brunswick Avenue
Indianapolis Indiana 46227(US)

(74) Representative: Crowther, Terence Roger et al,
Erl Wood Manor
Windlesham Surrey GU20 6PH(GB)

(54) **Process for preparing 5-substituted pyrimidines.**

(57) The present invention relates to a process for preparing a 5-substituted pyrimidine by the nucleophilic displacement of a 5-halopyrimidine under pressure for short periods of time.

EP 0 139 477 A2

Croydon Printing Company Ltd.

# PROCESS FOR PREPARING 5-SUBSTITUTED PYRIMIDINES

This invention relates to a novel process for preparing 5-substituted pyrimidine compounds.

The unreactivity of 5-halopyrimidines toward nucleophilic displacement is well known in the art. See, e.g., The Chemistry of Heterocyclic Compounds, D. J. Brown, The Pyrimidines Chapter IX, C, Interscience Publishers (1962). Typically, displacement of the halogen atom at the 5-position of the pyrimidine ring is carried out at standard pressure and high temperatures for long periods of time oftentimes with only moderate success. The process of the present invention relates to a convenient and economical procedure for the nucleophilic displacement of 5-chloro- and 5-bromopyrimidine analogs. Certain advantages of the present process include a much shorter reaction time as well as the ability to conduct the displacement at much lower temperatures than previously known. Further, yield increases are apparent depending on the nucleophile employed in the synthesis.

More specifically the present invention relates to a novel process for preparing a 5-substituted pyrimidine of the formula

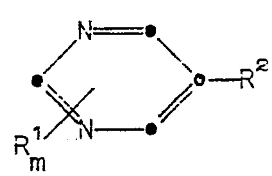

I

wherein $R^1$ is $C_1$-$C_6$ alkyl or phenyl, $R^2$ is $-NH_2$, $-NHCH_3$, $-N(CH_3)_2$ or $C_1$-$C_6$ alkoxy and m is 0-3, comprising contacting a 5-halopyrimidine of the formula

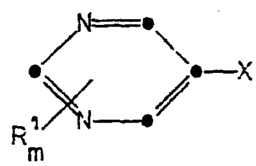

II

wherein X is bromo or chloro and $R^1$ and m are as defined above, with an appropriate nucleophile in the presence of a catalytically sufficient amount of a copper catalyst under pressure at a temperature in the range of from about 70°C to about 200°C for a period of approximately 10 to 90 minutes.

The term $C_1$-$C_6$ alkyl, as used herein, relates to a straight chain or branched alkyl chain having from one to six carbon atoms. Typical $C_1$-$C_6$ alkyl groups include methyl, ethyl, n-propyl, isopropyl, n-butyl, sec-butyl, t-butyl, isobutyl, n-pentyl, isopentyl, n-hexyl, and the like.

$C_1$-$C_6$ Alkoxy is a straight or branched alkoxy chain having from one to six carbon atoms. Exemplary $C_1$-$C_6$ alkoxy groups are methoxy, ethoxy, n-propoxy, n-butoxy, neopentoxy, n-hexoxy and the like.

While the entire scope of process variables taught herein are believed advantageous, the present process does have preferred aspects. Preferably, as designated in the above formula, X is bromine, m is 0

and $R^2$ is $-NHCH_3$, $-N(CH_3)_2$ or especially $-NH_2$ Other preferred process conditions will be noted hereinafter.

The process of this invention is conducted by contacting a 5-halopyrimidine with an appropriate nucleophile according to the conditions outlined below. The term "appropriate nucleophile", as defined herein, relates to a reagent capable of displacing the halogen atom from the pyrimidine starting material (II) to yield a product of formula I. Typical of these nucleophiles are ammonia, monomethylamine, dimethylamine or an alkali metal $C_1$-$C_6$ alkoxide derivative. Typical alkali metals are lithium, potassium and preferably sodium. These alkali metal $C_1$-$C_6$ alkoxide reagents are either commercially available or readily prepared by combining the desired alkali metal with an appropriate alcohol under standard reaction conditions. The nitrogen containing nucleophiles are all commercially available.

The quantity of nucleophile present in the reaction mixture is not critical and typically an excess amount is employed. When an amine is used as the nucleophile, generally a sufficient amount of the amine is used to account for the hydrochloric or hydrobromic acid salt formed thereby. Accordingly, from one to ten equivalents of the nucleophilic amine is typically employed in the present process. Water is the solvent of choice in this reaction, although other protic solvents such as the alcohols may be used. When an alkali metal $C_1$-$C_6$ alkoxide derivative is employed as the nucleophile, methanol or ethanol are the preferred

solvents and similar quantities of nucleophile may be used.

A catalytically sufficient amount of a copper catalyst is also necessary for the process of the present invention. The term "catalytically sufficient amount", as defined herein, relates to an amount of copper catalyst which is capable of increasing the rate of the reaction. The quantity of catalyst present in the reaction mixture is typically relatively small in comparison to the quantity of reactants and may be present from approximately 0.01% to about 15.0% by weight of catalyst to the weight of the starting 5-halopyrimidine (II). While larger amounts of catalyst may be employed if desired, they appear not to contribute to the operability of the reaction. While any of the copper reagents may be employed as catalysts, such as copper metal, cuprous chloride, cupric chloride or cupric nitrate, cupric sulfate is preferred.

The present process is conducted in a reaction vessel or autoclave capable of sustaining high pressures. The type of reaction vessel is not critical to the operability of the reaction. While glass or resin autoclaves will sustain pressures up to approximately 60 psi, stainless steel vessels are preferred because of their ability to sustain much higher pressures. The reaction ingredients are added to the autoclave so as to leave an air pocket in the vessel when it is sealed. Several vessels come equipped with a means of agitating the reaction ingredients, and this is preferably carried

out. A heat source, such as a thermal jacket, is also applied to the reaction vessel.

The reaction is preferably carried out at a temperature in the range of from about 70°C to about 175°C, more preferably between 100°C and 150°C, especially at approximately 140°-145°C. As the temperature rises in the reaction vessel, the pressure increases as well, according to the formula

$$P = \frac{nRT}{V}$$

wherein n, the number of moles of the 5-halopyrimidine, R, the gas constant, and V, the volume of the reaction vessel employed, are fixed values. Therefore as the temperature (T) is increased the pressure (P) increases proportionately. Typical pressures are in the range of about 30 psi to about 400 psi depending on the parameters outlined above.

When the process is carried out as defined above, the reaction will be complete in a relatively short period of time, typically from about 10 minutes to 90 minutes after the temperature of the reaction mixture reaches the desired range. Surprisingly, it has been observed that a longer reaction time actually reduces the amount of product obtained. For example, when conducted at the preferred temperature of about 140°-145°C and pressure of 200 psi, the reaction is substantially complete after about 30 minutes. The product is then isolated by standard procedures. Typically, the reaction mixture is either evaporated to

dryness under reduced pressure and extracted, or if water is used as the reaction solvent, extracted with a water immiscible organic solvent. The organic phase is then evaporated to dryness and the residual product thus formed further purified if desired by crystallization from common solvents or purification over solid supports such as silica gel or alumina.

The compounds prepared by the process of the present invention are useful for a variety of purposes. For example, U.S. Patent, 4,323,570 discloses certain substituted 5-aminopyrimidine derivatives as intermediates to compounds useful for the treatment of hypertension. Our pending Application No. 84304094.0 teaches the alkylation of certain of the aminopyrimidines presently prepared to provide compounds having fungicidal activity. The 5-alkoxypyrimidines prepared by the present process may be dealkylated by standard procedures and the hydroxy derivatives thus prepared may be esterified to provide compounds having insecticidal and acaricidal activity. See, e.g., U.S. Patent No. 4,127,652. The N,N-dimethyl-5-pyrimidinamines prepared by the present process are useful for controlling coccidiosis in animals whereby the compound is preferably orally administered in the feedstuff of the diseased animal. The active agent will kill or severely inhibit the growth of the coccidiosis causing organism when administered at a rate in the range of from about 1 to about 1000 ppm., more preferably from about 10 to 400 ppm. of the dimethylamine.

The 5-halopyrimidine compounds used as starting materials for the process of the present invention are either commercially available or readily prepared by known procedures. For example, U. S. Patent No. 4,252,955 teaches the reaction of formamide at high temperatures with a 4-halo-5-hydroxy-2(5H)furanone to provide 5-chloro- or 5-bromopyrimidine. For a general overview of the synthesis of the starting materials employed in the present process, see The Chemistry of Heterocyclic Compounds, D. J. Brown The Pyrimidines, Chapter VI, No. 2, Interscience Publishers (1962).

The process of the present invention is further illustrated by the following examples. These examples are not intended to be limiting in any respect and should not be so construed.

Example 1

Preparation of 5-aminopyrimidine

A mixture of 79.5 g (0.5 mol) of 5-bromopyrimidine, 250 ml of concentrated ammonium hydroxide and 5 g (0.031 mol) of cupric sulfate was placed in a 500 ml stainless steel autoclave (Autoclave Engineers, Erie, Pennsylvania). The autoclave was sealed and a heating jacket was placed around it. The reaction mixture was agitated with the stirrer turning at approximately 200 rpm and was allowed to warm gradually until after 29 minutes the temperature of the reaction vessel was 142°C and the pressure was 200 psi. The solution

was reacted at this temperature for approximately 15 minutes during which time the pressure dropped to 150 psi. The heat was removed and when the aqueous mixture had sufficiently cooled it was extracted three times with 100 ml portions of diethyl ether. The organic extracts were combined, dried and concentrated to provide 3.3 g of pale yellow crystals. This material was analyzed by nmr to show that it contained a 47/53 ratio of starting material to product. The aqueous phase was divided into two 150 ml portions. The first portion was placed on a continuous liquid-liquid extractor employing chloroform as the solvent. After approximately 16 hours, a tan solid was collected by filtration from the chloroform extracts to provide 14.3 g of 5-aminopyrimidine. The mother liquor from the collected solid was concentrated to dryness under reduced pressure to provide 5.7 g of crude 5-aminopyrimidine. The 5.7 g thus collected was slurried in approximately 20 ml of diethyl ether, filtered, washed with diethyl ether and air dried to provide 4.43 g of pure 5-aminopyrimidine. The other 150 ml aqueous aliquot was placed on the continuous chloroform extractor for approximately 60 hours. Upon cooling, the crystallized product was collected by filtration to afford 14.9 g of 5-aminopyrimidine. The mother liquor was again concentrated to dryness and slurried in diethyl ether to provide 3.86 g of product. Accordingly, a total of 37.49 g of 5-aminopyrimidine was isolated (79% yield). mp = 170°-171°C.

Example 2

Preparation of N,N-dimethyl-5-pyrimidinamine

A mixture of 39.7 g (0.25 mol) of 5-bromopyrimidine, 2.5 g (0.016 mol) of anhydrous cupric sulfate, 210.9 g (1.875 mol) of 40% aqueous dimethylamine and enough water to bring the total volume of the reaction mixture to 380 ml was placed in a 500 ml stainless steel reaction vessel. This vessel was sealed and heated to approximately 113°C and 60 psi for 30 minutes. The cooled reaction mixture was filtered and the filtrate was extracted three times with 100 ml portions of diethyl ether. The organic extracts were combined, dried and evaporated to dryness under reduced pressure to afford 16.9 g of starting material and N,N-dimethyl-5-pyrimidinamine in a ratio of 95:5. The aqueous phase was placed on the continuous extractor employing chloroform as the solvent for approximately 16 hours. The organic phase was separated, filtered and evaporated to provide 12.4 g of an amber clear oil. The aqueous phase was again placed on a continuous extractor for 4 more hours to provide an additional 0.8 g of oil. The two oily extracts were combined and distilled *in vacuo* at a pressure of from 2 to 3 mm. The fraction boiling from 97° to 103°C was collected to provide 2.4 g (7.8% yield) of N,N-dimethyl-5-pyrimidinamine.

Example 3

Preparation of N-methyl-5-pyrimidinamine

A solution of 80 g (0.5 mol) of 5-bromopyrimidine, 290 g (3.74 mol) of 40% aqueous monomethylamine and 5 g (0.031 mol) of cupric sulfate was added to an autoclave reaction vessel. The reaction mixture was heated to approximately 140°C for one hour with a starting pressure of 108 psi and an ending pressure of 131 psi. The reaction mixture was cooled to room temperature and placed on the continuous extractor for 16 hours employing chloroform as the solvent. The precipitated solid was collected by filtration and recrystallized from toluene to provide 22.3 g of N-methyl-5-pyrimidinamine. A small portion of this material was recrystallized from toluene to provide tan needles (mp = 95°-98°C). The aqueous phase was again extracted continuously with chloroform for approximately 16 hours and the organic phase was concentrated under reduced pressure to provide 23.7 g of brown solid. The total yield was 50.5 g which represents a yield of 92.7% product. The small portion of product that was recrystallized from the first extraction was further purified employing a silica gel column and eluting with methanol. The material that was collected from the column was dissolved in a small amount of hot toluene, the solution was cooled and the precipitated solid was collected by filtration to give tan crystals of N-methyl-5-pyrimidinamine. mp = 99°-100.2°C.

Analysis calculated for $C_5H_7N_3$

Theory: C, 55.03; H, 6.74; N, 38.50;

Found : C, 54.78; H, 6.23; N, 38.20.

## Example 4

### Preparation of 2-t-butyl-5-aminopyrimidine

A solution of 1.35 g (0.006 mol) of 2-*t*-butyl-5-bromopyrimidine, 0.15 g (0.94 mmol) of cupric sulfate and 200 ml of concentrated ammonium hydroxide was reacted under agitation in an autoclave at 150°C for approximately one hour and 25 minutes. The pressure of the reaction at this point was 250 psi. The reaction mixture was subsequently cooled and 3 ml of 50% sodium hydroxide was added to clear the solution. This material was extracted three times with 70 ml portions of ethyl acetate and the organic extracts were combined and dried over anhydrous sodium sulfate. Evaporation under reduced pressure gave 0.95 g of a light brown solid. This material was recrystallized from hexane/ethyl acetate (90/10) to give 0.57 g of product. mp = 95°-105°C. This material was eluted through a small silicone dioxide plug eluting with ethyl acetate/hexane (1/1) and 0.44 g of material was obtained. mp = 133°-136°C. The mother liquor was eluted through a 50 g silicone dioxide column with ethyl acetate to provide an additional 0.04 g of product. The total purified yield was 0.48 g (50% yield) of 2-*t*-butyl-5-pyrimidineamine.

## Example 5

### Preparation of 5-Methoxypyrimidine

A solution of 6.32 g (0.275 mol) of sodium metal dissolved in 300 ml. of methanol was added to a solution of 40 g. (0.25 mol.) of 5-bromopyrimidine and 2.5 g (0.016 mol.) of cupric sulfate in 100 ml. of methanol. The mixture was charged into a 500 ml. autoclave reaction vessel. The sealed vessel was heated at approximately 144°C. for one hour. The initial pressure was 170 psi and the final pressure was 180 psi. The mixture was cooled and evaporated to dryness under reduced pressure. The residue was dissolved in diethyl ether, filtered, and concentrated under vacuum to provide 23.4 g of 5-methoxypyrimidine (85% yield). The residue was further purified by crystallization from hexane to provide 17.88 g of 5-methoxypyrimidine (65% yield). mp = 45°-47°C.

## CLAIMS

1. A process for preparing a 5-substituted pyrimidine of the formula

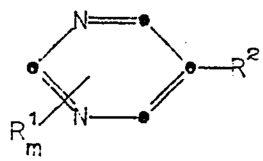

I

wherein $R^1$ is $C_1$-$C_6$ alkyl or phenyl, $R^2$ is $-NH_2$, $-NHCH_3$, $-N(CH_3)_2$ or $C_1$-$C_6$ alkoxy and m is 0-3, comprising contacting a 5-halopyrimidine of the formula

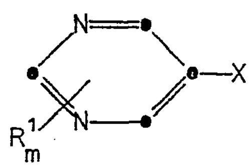

II

wherein X is bromo or chloro and $R^1$ and m are as defined above, with an appropriate nucleophile in the presence of a catalytically sufficient amount of a copper catalyst under pressure at a temperature in the range of from about 70°C to about 200°C for a period of approximately 10 to 90 minutes.

2. A process of Claim 1 wherein $R^2$ is $-NH_2$.

3. A process of Claim 1 wherein X is bromo.

4. A process of Claim 1 wherein the copper catalyst is cupric sulfate.

5. A process of Claim 1 wherein m is 0.

6. The process of Claim 5 wherein the compound of formula I is 5-aminopyrimidine.

7. The process of Claim 5 wherein the compound of formula I is N,N-dimethyl-5-pyrimidinamine.

8. The process of Claim 5 wherein the compound of formula I is N-methyl-5-pyrimidinamine.

9. The process of Claim 5 wherein the compound of formula I is 2-t-butyl-5-aminopyrimidine.

10. The process of Claim 5 wherein the compound of formula I is 5-methoxypyrimidine.